# EUROPÄISCHE PATENTSCHRIFT

(11) **EP 1 283 692 B1**
(45) Veröffentlichungstag und Bekanntmachung des Hinweises auf die Patenterteilung: **04.05.2005**
(21) Anmeldenummer: 01943089.1
(22) Anmeldetag: 16.05.2001
(51) Int. Cl.: A61F 2/08

(54) **VERBUND EINES ALLOPLASTISCHEN TEXTILBANDES MIT ZWEI FIXATIONSELEMENTEN**
COMPOSITE CONSISTING OF AN ALLOPLASTIC TEXTILE BAND WITH TWO FIXING ELEMENTS
ENSEMBLE CONSTITUE D'UNE BANDE TEXTILE ALLOPLASTIQUE COMPORTANT DEUX ELEMENTS DE FIXATION

(30) Priorität: 16.05.2000 DE 10023680
(43) Veröffentlichungstag der Anmeldung: 19.02.2003
(73) Patentinhaber: Freundes- und Förderkreis des Institutes für Textiltechnik der RWTH Aachen e.V., 52062 Aachen (DE); Marx, Rudolf, 53533 Eichenbach (DE); Fischer, Horst, 52074 Aachen (DE); Erli, Hans Josef, 4720 Kelmis (BE)
(72) Erfinder: MARX, Rudolf, 53533 Eichenbach (DE); FISCHER, Horst, 52074 Aachen (DE); ERLI, Hans, Josef, 4720 Kelmis (BE); BERNDT, Erik, 52064 Aachen (DE)
(74) Vertreter: König, Werner, Dipl.-Ing.
(86) Internationale Anmeldenummer: PCT/DE2001/001854
(87) Internationale Veröffentlichungsnummer: WO 2001/087185

(56) Entgegenhaltungen:
- US-A- 4 364 731
- US-A- 4 590 928

## Beschreibung

Die Erfindung betrifft einen Verbund eines alloplastischen Textilbandes, insbesondere als Ligament- oder Sehnenersatz, mit zwei an den Enden des Textilbandes vorgesehenen Fixationselementen, die an je einem Knochen bzw. an einem Knochen und an einem Ligament oder einer Sehne festgelegt sind.

Aus der EP 0 260 787 A1 ist ein alloplastischer Ligamentersatz bekannt, bei dem ein alloplastisches Textilband mittels zweier Fixationselemente am Knochen befestigt ist. Dabei ist das Textilband in einer Ausführungsform derartig ausgebildet, dass es endseitig jeweils mit einer augenartigen Bandschlinge ausgeführt ist. In ein Bandschlingenauge greift ein zum Fixationselement gehöriger Gelenkzapfen ein. Nachteilig an dieser Ausführung ist, dass das in den Gelenkzapfen eingehängte Bandende bei Zug unregelmäßig beansprucht wird. Bei Drehbewegungen ist das Bandende empfindlich gegenüber Abrasion. Zudem muss im Bereich der Bandschlinge eine ausreichende Banddicke gewählt werden, damit das Textilband der Zugbelastung standhalten kann. Des Weiteren ist die Flechtung der Bandenden aufwendig und somit kostenintensiv.

Bei einer weiteren Ausführungsform eines Bandendes als Bandauge wird das Bandende mittels einer Schraube am Knochen fixiert. Dabei wird das Bandende gequetscht, was zu einer deutlichen Minderung der Zugfestigkeit führen kann.

In einer weiteren Ausführungsform ist das Bandende als kugelförmige Verdickung ausgeführt. Diese Kugel wird in einer zu einem Fixationselement gehörigen künstlichen Gelenkpfanne geführt. Hier entspricht die Banddicke nicht der des nativen Bandes. Zudem wird das Bandende in der künstlichen Gelenkpfanne abrasiv belastet. Auch ist die kugelförmige Ausbildung der Bandenden aufwendig und somit kostenintensiv.

Aus der DE 37 10587 C2 ist ebenfalls ein alloplastischer Ligamentersatz bekannt, bei dem ein Textilband mittels zweier Fixationselemente am Knochen befestigt ist und die Bandenden endseitig mit augenartigen Bandschlingen drehgelagert ausgeführt sind. Für diese Ausführungsform gelten die bereits oben genannten Nachteile für Textilbänder mit augenartigen Bandschlingen fort.

Aus der US 3 988 783 ist eine Ligamentprothese mit einem bügelartigen Verbindungselement und zwei Befestigungselementen zur Befestigung an Knochen offenbart. Das Verbindungselement ist an seinen beiden Enden mit jeweils einem Auge ausgebildet, in das ein mit dem Befestigungselement verbundener Bolzen zugkraftaufnehmend eingreift. Es *kann zusätzlich* zu dieser Bolzenbefestigung eine Verklebung eines Verbindungselementes mit einem Befestigungselement vorgesehen sein. Hiermit soll *nicht* die Herauslösung des Verbindungselement-Auges aus dem Befestigungselement-Bolzen, sondern lediglich eine Schwenkbewegung zwischen Befestigungs- und Verbindungselement verhindert werden. Dabei wird der Kleber zwischen den beiden Elementen einer Scherbelastung ausgesetzt. Eine Zugbelastung hat der Kleber hierbei nicht aufzunehmen. Die Augen-Bolzenverbindung stellt eine platzfordernde Verbindung dar. Es sollen mit der vorgestellten Ligamentprothese zwei Knochen miteinander verbunden werden. Eine Festlegung an einem Ligament bzw. einer Sehne und/oder an einen Knochen ist nicht möglich.

In der US 4 364 731 ist ein Verfahren zum Verblenden von Zahnkronen offenbart, bei dem eine konditionierte Substratoberfläche mit einer Polymerverblendung fest verbunden werden soll. Zur Konditionierung wird auf die mit einfachen Mitteln gereinigte Substratoberfläche eine Silikatschicht aufgesputtert und auf diese ein Silanhanftvermittler aufgebracht. Auf die derart konditionierte Schicht wird dann die Verblendung aufpolymerisiert. Diese Verblendung ist als abschließende Schicht ausgebildet, d.h. sie soll nur zu der konditionierten Seite hin eine Verbindung aufweisen. Zu ihrer freien Seite hin soll die Endschicht inert sein, d.h. eine Reaktion der freien Seite der Verblendung mit Fremdstoffen, z.B. ein Verkleben mit Essensresten auf die Verblendung, ist unerwünscht. Diese Verblendung muss keine Zugkräfte aufnehmen.

Die Aufgabe der vorliegenden Erfindung besteht nun darin, ein alloplastisches Textilband als Ligament- (insbesondere Kreuzband-) oder Sehnenersatz zu schaffen und dieses mit Hilfe von Fixationselementen in möglichst originärer physiologischer Lage am Knochen, an einem Ligament oder an einer Sehne derart zu befestigen, dass es bei Zugbeanspruchung eine möglichst gleichmäßige, physiologische Belastung über den Befestigungsquerschnitt erfährt.

Zur Lösung dieser Aufgabe ist erfindungsgemäß bei einem Verbund der eingangs angegebenen Art vorgesehen, dass das Textilband und zumindest eines der Fixationselemente ausschließlich mittels eines Adhäsivs zugkraftaufnehmend miteinander verklebt sind. Es kann auf weitestgehend plane Bänder, die der Form natürlicher Bänder nachempfunden sind, zurückgegriffen werden. Es sind Standardgrößen für die Bänder vorgesehen, deren Längenfeinabstimmung durch eine in eines der Fixationselemente integrierte Stellvorrichtung erfolgen soll. Eine Verklebung mit dem Fixationselement ergibt bei Belastung des Bandes durch Zug eine gleichmäßige Bandbeanspruchung. Schwächezonen, wie sie z.B. bei Bandschlingen gegeben sind, entfallen. Somit kann eine längere Standzeit des Bandersatzes erreicht werden. Ist ein Textilband mit nur einem zu verklebenden Fixationselement vorgesehen, so ist als zweites Fixationselement eine Naht an einem Ligament- oder Sehnenrest vorstellbar. Als zu verklebende Fixationselemente sind übliche, aber an die hier vorliegende Technologie adaptierte Fixationselemente einzusetzen.

In den Fixationselementen kann jeweils ein kugelförmiger Anschlusskörper drehbar gelagert sein, mit dem das Kunststoffband zugkraftaufnehmend mittels eines Adhäsivs verbunden ist. Auf diese Weise kann sich das Kunststoffband ohne zusätzliche Belastungen etwaigen Änderungen des Bandverlaufs anpassen.

Die Fixationselemente können z. B. als Stellelement mit einer Stellschraube ausgebildet sein. Auch kann das Fixationselement eine flache Hülse sein, in die das Bandende einzuführen ist. Es ist vorstellbar, aber nicht erfindungsgemäß, zur Unterstützung der Klebung Klemmelemente einzusetzen. Die Fixationselemente können je nach Bedarf aus resorbierbaren Kunststoffen und/oder nicht resorbierbaren polymeren Kunststoffen (z. B. Polycarbonat, Bis-GMA, PMMA), resorbierbaren Keramiken oder Gläsern oder nicht resorbierbaren Keramiken oder Gläsern (hochbelastbare Strukturkeramiken wie z.B. Aluminium- oder Zirkonoxid) oder Metall (z.B. CoCr- oder Ti-Legierungen bzw. Titan) hergestellt sein. Selbstverständlich sind auch noch weitere Werkstoffe vorstellbar.

Die diesseits beanspruchten alloplastischen Textilbänder und Fixationselemente werden gegebenenfalls an unterschiedlichen Hightech-Produktionsstätten angefertigt und dann separat an die Verbraucher, d. h. Krankenhäuser im In- und Ausland, versendet. Dort lagern die Einzelteile gegebenenfalls einige Monate bevor sie operativ eingesetzt werden. Über diesen gesamten Zeitraum, der durchaus sechs bis zwölf Monate umspannen kann, muss die Sterilität von Bändern und Fixationselementen garantiert werden. Für diesen Fall ist es vorstellbar, als Adhäsiv ein Mehrkomponentenadhäsiv, insbesondere ein polymeres Mehrkomponentenadhäsiv, einzusetzen. Hierbei kann die erste Komponente bereits an den Produktionsstätten auf Bandenden und/oder Fixationselement(e) als sterile und/oder nach der Auspolymerisation sterilisierbare konservierende Schutzschicht aufgebracht werden. Beim Einsatz im Operationssaal kann dann die erste Komponente mit einer zweiten Adhäsivkomponente, z.B. einem sterilen MMA-Monomer, bestrichen und dadurch aktiviert werden. Nach dem Aufbringen einer dritten Adhäsivkomponente, einer polymeren Komponente, werden die derart präparierten Fixationselemente und Bandenden miteinander in Verbindung gebracht und somit verklebt. Selbstverständlich sind auch Mehrkomponentenadhäsive mit nur zwei oder mehr als drei Komponenten vorstellbar. Nach der Verfestigung ist eine Verbindung hoher und langzeitstabiler Haftfestigkeit entstanden.

Erfindungsgemäß ist ferner vorgesehen, dass das Textilband und/oder mindestens ein Fixationselement jeweils zumindest partiell mit einer Silikatschicht und darüber mit einem Silanhaftvermittler versehen sein können und zwischen den so konditionierten Oberflächen eine Adhäsivschicht vorhanden sein kann. Dabei kann die Silikatschicht auf die Fixationselemente im Hochvakuum aufgedampft werden, und auf das Textilband können durch tribochemische Implantation silikatbeschichtete Korundkörper aufgebracht werden. Durch einen derartigen Schichtaufbau wird eine hydrolysestabile Verbindung zwischen Textilband und Fixationselement erreicht.

Erfindungsgemäß kann ferner vorgesehen sein, dass das Textilband (1) und/oder mindestens ein Fixationselement (2,3) jeweils zumindest partiell plasmabehandelt sind. Die Plasmabehandlung sollte vor dem Aufbringen der Silikatschicht erfolgen..

Des Weiteren ist erfindungsgemäß vorgesehen, dass das Textilband mit zumindest einer ebenen oder gewölbten inneren oder äußeren Mantelfläche eines Fixationselementes verklebt sein kann. Bei einer bevorzugten Ausführungsform ist das Fixationselement hülsenförmig ausgebildet und das Textilband mit den Innenflächen des Fixationselementes verklebt. Die Innenflächen können dabei mit Erweiterungen oder Einengungen versehen sein. Zur Vergrößerung der Oberfläche des Textilbandes im Bereich des mit einem Fixationselement zu verbindenden Endes können die Fasern des Textilbandes hier ganz oder zum Teil büschelartig nebeneinander verlaufen.

Erfindungsgemäß ist ferner vorgesehen, dass das Textilband ein Geflecht, ein Gewebe oder eine Maschenware sein kann. Dabei können auch dreidimensional geflochtene Kunstbänder eingesetzt werden. Das Textilband kann erfindungsgemäß ferner eine Kombination verschiedener Textilstrukturen sein. Es kann auch aus einer Kombination verschiedener Fasern bestehen.

Des Weiteren ist erfindungsgemäß vorgesehen, dass das Textilband aus Synthesefasern wie Polypropylen, Polyester, Polyvinylidenfluorid, Polytetrafluorethylen, Polyetherteherketon und/oder aus Metall-, Glas-, Keramik- oder Carbonfasern bestehen kann. Für den Kreuzbandersatz haben sich insbesondere Textilbänder aus Polyester und Polyvinylidenfluorid bewährt.

Ferner kann erfindungsgemäß vorgesehen sein, dass das Textilband zumindest teilweise aus mindestens zum Teil resorbierbaren Materialien besteht. Dabei kann es sich z.B. um resorbierbare polymere Kunststoffe oder um resorbierbare Gläser oder resorbierbaren Keramiken handeln. Resorbierbare Bänder können als Einheilungshilfe fungieren.

Erfindungsgemäß ist ferner vorgesehen, dass die Oberfläche des Textilbandes durch Silikat tragende Korundkörner und/oder eine silikatische Beschichtung mit Silikat angereichert sein kann. Die Korundkörner werden in einem Strahlprozess in die Bandoberfläche tribochemisch implantiert, wobei die Retention der Körner in der Oberfläche chemischer oder rein mechanischer Art sein kann. Durch die Implantation der Korundkörner wird die Haftfestigkeit zwischen Textilband und Silanhaftvermittler deutlich heraufgesetzt. Eine silikatische Beschichtung des Textilbandes kann bei einigen Werkstoffen auch durch Aufdampfen erfolgen.

Erfindungsgemäß ist ferner vorgesehen, dass mindestens ein Fixationselement zumindest teilweise aus mindestens zum Teil resorbierbaren Materialien bestehen kann. Resorbierbare Fixationselemente können zusammen mit resorbierbaren Bändern als Einheilungshilfe fungieren.

Des Weiteren ist erfindungsgemäß vorgesehen, dass als Silanhaftvermittler ein 3-Methacryloxypropyltrimethoxysilan vorgesehen sein kann. Selbstverständlich sind auch weitere Silanhaftvermittler vorstellbar.

Erfindungsgemäß ist ferner vorgesehen, dass das Adhäsiv ein körperverträgliches Adhäsiv sein kann. Hierbei können insbesondere Bis-GMA oder PMMA vorgesehen sein. Bis-GMA wird bisher aufgrund seiner hervorragenden Körperverträglichkeit bevorzugt im medizinischen Bereich als Dentaladhäsiv oder als Knochenzement eingesetzt.

Schließlich ist erfindungsgemäß vorgesehen, dass der Verbund als Implantat oder Prothese bzw. als Komponente von einem Implantat oder einer Prothese zu verwenden ist. Dabei ist bevorzugt an einen Einsatz als Ligament- oder Sehnenersatz zu denken.

Die Erfindung soll nachstehend anhand eines Ausführungsbeispiels näher erläutert werden. In den zugehörigen Zeichnungen zeigen:
- Fig. 1: einen Schnitt durch ein alloplastisches Textilband mit zwei Fixationselementen,
- Fig. 2: eine schematische Ansicht eines Endes eines Textilbandes und
- Fig. 3: eine schematische Darstellung eines Fixationselementes.

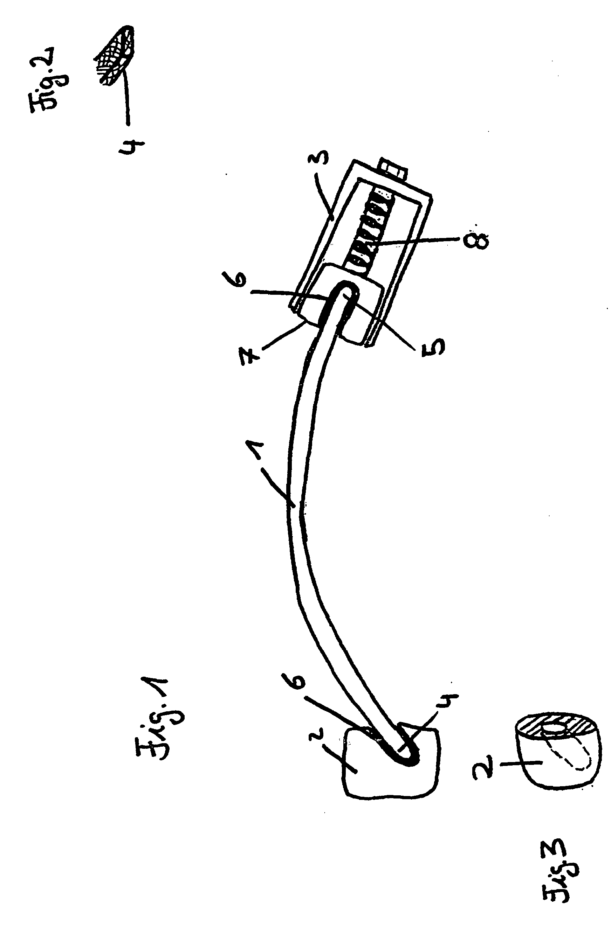

Figur 1 zeigt einen Schnitt durch ein alloplastisches Textilband 1 mit zwei Fixationselementen 2,3 zur Verwendung als Kreuzbandersatz. Auf eine Darstellung der Knochen, an denen die Fixationselemente 2,3 befestigt werden, wurde verzichtet.

Das alloplastische Textilband 1, z.B. ein geflochtenes Textilband aus Polyester, ist mit jedem seiner Enden 4,5 mit jeweils einem Fixationselement 2,3 verklebt. Hierzu werden sowohl das Textilband 1 als auch die Fixationselemente 2,3 jeweils zumindest partiell plasmabehandelt und mit einer Silikatschicht versehen. Dabei wird das Silikat in die Textilbandoberfläche durch tribochemische Implantation mit Silikat beschichteter Korundkörner eingebracht. Auf das Fixationselement 2,3 kann die Silikatschicht aufgedampft werden. Auf die Silikatschicht von Textilband 1 und Fixationselement 2,3 wird ein Silanhaftvermittler, z.B. 3-Methacryloxypropyltrimethoxysilan, aufgebracht. Zwischen den so konditionierten Oberflächen von Textilband 1 und Fixationselement 2,3 wird dann das körperverträgliche Adhäsiv 6 aufgebracht. Als Adhäsiv 6 dient z.B. ein PMMA. Durch die Verklebung wird eine optimale Befestigung des Textilbandes 1 über den Befestigungsquerschnitt erzielt, so dass bei Zug die Beanspruchung des Textilbandes 1 gleichmäßig über den Befestigungsquerschnitt erfolgt. Durch die Verklebung kann die Zugkraft vollständig von den Bändern auf die Fixationselemente übertragen werden.

Als Fixationselemente 2,3 sind handelsübliche Fixationselemente 2,3 , z.B. aus Titan, nach dem Aufbringen oben beschriebener Beschichtung geeignet. Dabei kann die Beschichtung auch an den Fixationselementoberflächen angebracht sein, an denen das Fixationselement 2,3 nicht mit dem Textilband verklebt ist Dies kann die Körperverträglichkeit des Fixationselementes 2,3 heraufzusetzen. Das erste Fixationselement 2 ist als einfache Hülse ausgebildet, in die ein Textilbandende 4 eingesetzt und verklebt ist. Bei dem zweiten Fixationselement 3 handelt es sich um ein Stellelement. Hier wird das zweite Textilbandende 5 in eine Stellhülse 7 mit Stellschraube 8 eingesetzt und verklebt Durch das Stellelement kann das Textilband 1 optimal eingespannt werden und bei Bedarf nach dem Einsetzen beim Patienten durch einen geringfügigen operativen Eingriff nachgestrafft werden. Gegebenenfalls kann die Verklebung von Fixationselement 2,3 und Textilband 1 durch die Ausbildung von zusätzlichen Retentionshilfen, z.B.Noppen, auf der Oberfläche des Fixationselementes 2,3 noch verbessert werden. Es ist auch vorstellbar, dass die Zugkraftaufnahme durch eine Kombination von einer Verklebung und einer Befestigung mittels Krallen, die am Fixationselement angebracht sind, erfolgt. Hierzu kann das Fixationselement auch als Klemmelement ausgebildet sein.

Das erfindungsgemäße Textilband 1 ist auch als künstlicher Seitenbandersatz z.B. am Knie, Sprunggelenk oder Finger einsetzbar. Zudem kann das Textilband 1 auch als Sehnenersatz fungieren. Hierzu wird das Textilband 1 mit einem seiner Enden 4,5 mit einem ersten Fixationselement 2,3 verklebt und über dieses an einem Knochen befestigt. Am anderen Ende 4,5 des Textilbandes 1 dient eine Naht, die Textilband 1 und Sehne miteinander verbindet, als zweites Fixationselement. Sofern ein resorbierbares Textilband 1 vorgesehen ist, wird das Textilband 1 nach und nach durch körpereigenes Material ersetzt. Das Entsprechende gilt bei Verwendung resorbierbarer Fixationselemente.

Figur 2 zeigt schematisch ein Ende eines Textilbandes 1. Dieses ist überwiegend flach ausgebildet und kann somit nach der Verklebung mit dem Fixationselement Kräfte über die gesamte Breite des Textilbandes 1 aufnehmen. Durch die Verklebung kann die Zugkraft vollständig von den Bändern auf die Fixationselemente übertragen werden. Durch die flache Ausbildung kann das Textilband 1 in der ursprünglichen physiologischen Ligament- bzw. Sehnenlage am Knochen fixiert werden. Dadurch wird die Belastung des natürlichen Bandes weitestgehend nachempfunden.

Figur 3 zeigt schematisch ein einfaches hülsenförmiges Fixationselement 2. Dieses kann aus einer Vielzahl körperverträglicher Materialien hergestellt sein. Diese Hülse ist auf ihren inneren Mantelflächen plasmabehandelt, silikatisiert und anschließend mit einem Silanhaftvermittler versehen. Ein Textilbandende 4,5 kann nun unter Verwendung eines körperverträglichen Adhäsivs 6, z.B. PMMA, in die Hülse eingelegt und dort verklebt werden. Selbstverständlich sind eine Vielzahl anderer Ausführungsformen der Fixationselemente vorstellbar.

## Patentansprüche

1. Verbund eines alloplastischen Textilbandes (1), insbesondere als Ligament- oder Sehnenersatz,
mit zwei an den Enden (4,5) des Textilbandes vorgesehenen Fixationselementen (2,3), die an je einem Knochen bzw. an einem Knochen und an einem Ligament oder einer Sehne festlegbar sind,
wobei das Textilband (1) und zumindest eines der Fixationselemente (2,3) ausschließlich mittels eines Adhäsivs (6) zugkraftaufnehmend miteinander verbunden sind.

2. Verbund nach Anspruch 1, **dadurch gekennzeichnet, dass** das Textilband (1) und/oder mindestens ein Fixationselement (2,3) jeweils zumindest partiell mit einer Silikatschicht und darüber mit einem Silanhaftvermittler versehen sind und dass zwischen den so konditionierten Oberflächen eine Adhäsivschicht (6) vorhanden ist.

3. Verbund nach Anspruch 2, **dadurch gekennzeichnet, dass** das Textilband (1) und/oder mindestens ein Fixationselement (2,3) jeweils zumindest partiell plasmabehandelt sind.

4. Verbund nach einem der vorhergehenden Ansprüche, **dadurch gekennzeichnet, dass** das Textilband (1) mit zumindest einer ebenen oder gewölbten inneren oder äußeren Mantelfläche eines Fixationselementes (2,3) verklebt ist.

5. Verbund nach einem der vorhergehenden Ansprüche, **dadurch gekennzeichnet, dass** das Textilband (1) ein Geflecht, ein Gewebe oder eine Maschenware ist.

6. Verbund nach einem der vorhergehenden Ansprüche, **dadurch gekennzeichnet, dass** das Textilband (1) eine Kombination verschiedener Textilstrukturen ist.

7. Verbund nach einem der vorhergehenden Ansprüche, **dadurch gekennzeichnet, dass** das Textilband (1) aus einer Kombination verschiedenartiger Fasern besteht.

8. Verbund nach einem der vorhergehenden Ansprüche, **dadurch gekennzeichnet, dass** das Textilband (1) aus Synthesefasern wie Polypropylen, Polyester, Polyvinylidenfluorid, Polytetrafluorethylen, Polyetheretherketon und/oder aus Metall-, Glas-, Keramik- oder Carbonfasem besteht

9. Verbund nach einem der vorhergehenden Ansprüche, **dadurch gekennzeichnet, dass** das Textilband (1) zumindest teilweise aus mindestens zum Teil resorbierbaren Materialien besteht.

10. Verbund nach einem der Ansprüche 2 bis 9, **dadurch gekennzeichnet, dass** die Oberfläche des Textilbandes (1) durch Silikat tragende Korundkömer und/oder durch eine silikatische Beschichtung mit Silikat angereichert ist.

11. Verbund nach einem der vorhergehenden Ansprüche, **dadurch gekennzeichnet, dass** mindestens ein Fixationselement (2,3) zumindest teilweise aus mindestens zum Teil resorbierbaren Materialien besteht.

12. Verbund nach einem der Ansprüche 2 bis 11, **dadurch gekennzeichnet, dass** als Silanhaftvermittler ein 3-Methacryloxypropyltrimethoxysilan vorgesehen ist

13. Verbund nach einem der vorhergehenden Ansprüche, **dadurch gekennzeichnet, dass** als Adhäsiv (6) ein körperverträgliches Adhäsiv (6) vorgesehen ist.

14. Verbund nach Anspruch 13, **dadurch gekennzeichnet, dass** als körperverträgliches Adhäsiv (6) ein Bis-GMA und/oder PMMA vorgesehen ist.

## Claims

1. Composite consisting of an alloplastic textile band (1), in particular as ligament or tendon replacement, with two fixing elements (2, 3) which are provided at the ends (4, 5) of the textile band and which can each be secured to a bone or to a bone and to a ligament or a tendon, the textile band (1) and at least one of the fixing elements (2, 3) being connected to one another exclusively by means of an adhesive (6) in such a way as to take up tensile force.

2. Composite according to Claim 1, **characterized in that** the textile band (1) and/or at least one fixing element (2, 3) is/are in each case provided at least in part with a silicate layer and, over the latter, with a silane coupling agent, and **in that** an adhesive layer (6) is present between the surfaces so conditioned.

3. Composite according to Claim 2, **characterized in that** the textile band (1) and/or at least one fixing element (2, 3) is/are in each case subjected at least in part to plasma treatment.

4. Composite according to one of the preceding claims, **characterized in that** the textile band (1) is adhesively bonded to at least one plane or curved, inner or outer superficies surface of a fixing element (2, 3).

5. Composite according to one of the preceding claims, **characterized in that** the textile band (1) is a braid, a woven fabric or a knitted fabric.

6. Composite according to one of the preceding claims, **characterized in that** the textile band (1) is a combination of different textile structures.

7. Composite according to one of the preceding claims, **characterized in that** the textile band (1) is composed of a combination of different types of fibres.

8. Composite according to one of the preceding claims, **characterized in that** the textile band (1) is composed of synthetic fibres such as polypropylene, polyester, polyvinylidene fluoride, polytetrafluoroethylene, polyetheretherketone and/or of metallic fibres, glass fibres, ceramic fibres or carbon fibres.

9. Composite according to one of the preceding claims, **characterized in that** the textile band (1) is composed at least in part of at least partially absorbable materials.

10. Composite according to one of Claims 2 to 9, **characterized in that** the surface of the textile band (1) is fortified by silicate-supporting corundum granules and/or by a siliceous coating with silicate.

11. Composite according to one of the preceding claims, **characterized in that** at least one fixing element (2, 3) is composed at least in part of at least partially absorbable materials.

12. Composite according to one of Claims 2 to 11, **characterized in that** the silane coupling agent provided is a 3-methacryloxypropyltrimethoxysilane.

13. Composite according to one of the preceding claims, **characterized in that** the adhesive (6) provided is a biocompatible adhesive (6).

14. Composite according to Claim 13, **characterized in that** the biocompatible adhesive (6) provided is a Bis-GMA and/or PMMA.

## Revendications

1. Ensemble d'une bande textile alloplastique (1), notamment sous forme de ligament ou de tendon de remplacement,
comprenant deux éléments de fixation (2, 3) prévus aux extrémités (4, 5) de la bande textile, qui peuvent être fixés à un os respectif ou à un os et à un ligament ou un tendon,
la bande textile (1) et au moins l'un des éléments de fixation (2, 3) étant connectés l'un à l'autre exclusivement au moyen d'un adhésif (6) en recevant la force de traction.

2. Ensemble selon la revendication 1, **caractérisé en ce que** la bande textile (1) et/ou au moins un élément de fixation (2, 3) sont pourvus chacun au moins en partie d'une couche de silicate et pardessus celle-ci d'un promoteur d'adhésion à base de silane et **en ce que** l'on prévoit entre les surfaces ainsi conditionnées une couche d'adhésif (6).

3. Ensemble selon la revendication 2, **caractérisé en ce que** la bande textile (1) et/ou au moins un élément de fixation (2, 3) sont chacun au moins en partie soumis à un traitement au plasma.

4. Ensemble selon l'une quelconque des revendications précédentes, **caractérisé en ce que** la bande textile (1) est collée à au moins une surface d'enveloppe intérieure ou extérieure, plane ou courbe, d'un élément de fixation (2, 3).

5. Ensemble selon l'une quelconque des revendications précédentes, **caractérisé en ce que** la bande textile (1) est un produit tressé, tissé ou maillé.

6. Ensemble selon l'une quelconque des revendications précédentes, **caractérisé en ce que** la bande textile (1) est une combinaison de différentes structures textiles.

7. Ensemble selon l'une quelconque des revendications précédentes, **caractérisé en ce que** la bande textile (1) est constituée d'une combinaison de fibres de types différents.

8. Ensemble selon l'une quelconque des revendications précédentes, **caractérisé en ce que** la bande textile (1) se compose de fibres synthétiques comme du polypropylène, du polyester, du fluorure de polyvinylidène, du polytétrafluoréthylène, de la polyétheréthercétone et/ou de fibres de métal, de verre, de céramique ou de carbone.

9. Ensemble selon l'une quelconque des revendications précédentes, **caractérisé en ce que** la bande textile (1) se compose au moins en partie de matériaux au moins en partie résorbables.

10. Ensemble selon l'une quelconque des revendications 2 à 9, **caractérisé en ce que** la surface de la bande textile (1) est enrichie en silicate par des grains de corindon portant du silicate et/ou par un revêtement au silicate.

11. Ensemble selon l'une quelconque des revendications précédentes, **caractérisé en ce qu'**au moins un élément de fixation (2, 3) se compose au moins en partie de matériaux au moins en partie résorbables.

12. Ensemble selon l'une quelconque des revendications 2 à 11, **caractérisé en ce que** l'on prévoit comme promoteur d'adhésion à base de silane un 3-méthacryloxypropyltriméthoxysilane.

13. Ensemble selon l'une quelconque des revendications précédentes, **caractérisé en ce que** l'on prévoit comme adhésif (6) un adhésif (6) compatible avec le corps.

14. Ensemble selon la revendication 13, **caractérisé en ce que** l'on prévoit comme adhésif compatible avec le corps (6) un Bis-GMA et/ou du PMMA.
